# EUROPÄISCHE PATENTSCHRIFT

(11) **EP 0 517 698 B1**
(45) Veröffentlichungstag und Bekanntmachung des Hinweises auf die Patenterteilung: **04.08.1993**
(21) Anmeldenummer: 90915411.4
(22) Anmeldetag: 31.10.1990
(51) Int. Cl.: F04B 43/04

(54) **MIKROMINIATURISIERTE PUMPE**
MICRO-MINIATURISED PUMP
POMPE MICROMINIATURISEE

(30) Priorität: 27.02.1990 DE 4006152
(43) Veröffentlichungstag der Anmeldung: 16.12.1992
(73) Patentinhaber: FRAUNHOFER-GESELLSCHAFT ZUR FÖRDERUNG DER ANGEWANDTEN FORSCHUNG E.V., 80636 München (DE)
(72) Erfinder: RICHTER, Axel, D-8000 München 70 (DE)
(74) Vertreter: Schoppe, Fritz, Dipl.-Ing.
(86) Internationale Anmeldenummer: DE9000830
(87) Internationale Veröffentlichungsnummer: WO9113255

(56) Entgegenhaltungen:
- EP-A- 392 978
- GB-A- 1 542 799
- US-A- 3 963 380

## Beschreibung

Die vorliegende Erfindung betrifft eine mikrominiaturisierte Pumpe mit zumindest einem ersten und einem zweiten, übereinander angeordneten und miteinander verbundenen Pumpenkörper, wobei zumindest einer der Pumpenkörper wenigstens teilweise aus einem Halbleitermaterial besteht, und wobei beide Pumpenkörper elektrisch leitfähige Bereiche umfassen, die gegeneinander isoliert sind, nach dem Oberbegriff des Patentanspruchs 1.

Aus der Fachveröffentlichung A. Richter und H. Sandmaier "An Electrohydrodynamic Micropump", IEEE MEMS-90, 12. - 14. Februar 1990, Napa Valley, Californien, USA ist eine derartige mikrominiaturisierte Pumpe bekannt. Diese aus der genannten Fachveröffentlichung, die anläßlich einer Fachtagung verbreitet wurde, bekannte Mikropumpe entspricht der Mikropumpe, die in der älteren, nicht vorveröffentlichten Anmeldung P 39 25 749.5-32 der Anmelderin offenbart ist. Bei dieser bekannten mikrominiaturisierten Pumpe handelt es sich um eine elektrohydrodynamische Injektionspumpe, deren in Pumprichtung voneinander beabstandeten Pumpenkörper als gitterförmige Halbleiterelektroden mit Oberflächenmetallisierung ausgebildet sind. Bei dieser Pumpe wird von einer der beiden gitterförmigen Elektroden eine Ioneninjektion in das zu pumpende Gas oder die zu pumpende, nötigerweise isolierende Flüssigkeit vorgenommen. Die Relativbewegung der Ionen zu dem zu pumpenden Gas oder zu der zu pumpenden Flüssigkeit erzeugt eine Pumpwirkung, so daß diese bekannte elektrostatische Pumpe ohne bewegliche Teile auskommt. Jedoch eignet sich diese bekannte Pumpe nicht zum Pumpen oder Dosieren wässriger Lösungen oder sonstiger leitfähiger Medien.

Eine nach einem anderen Wirkungsprinzip arbeitende mikrominiaturisierte Pumpe ist in der Fachveröffentlichung F.C.M. van de Pol et al., "A Thermopneumatic Micropump Based on Micro-Engineering Techniques" des Konferenzberichtes "Transducers '89" The 5th International Conference on Solid-State Sensors and Actuators & Eurosensors III, Juni 25 - 30, 1989, Montreux, Schweiz, bekannt. Bei dieser bekannten Mikropumpe liegt ein Heizwiderstand in einer mit einem Gas gefüllten, durch eine Membran abgeschlossenen Kammer. Durch Aufheizen und Abkühlen des Heizwiderstandes kann das Gasvolumen erhöht und abgesenkt werden. Hierdurch kann die Membran der Kammer elektrisch betätigt werden. Auf der dem Heizwiderstand abgewandten Seite der Membran liegt eine Kammer mit der zu pumpenden Flüssigkeit, die einlaßseitig und auslaßseitig durch je ein Rückschlagventil abgeschlossen ist, wodurch eine Bewegung der Membran zu einer Pumpförderung der in der Kammer eingeschlossenen Flüssigkeit führt. Diese bekannte Mikropumpe ist aus fünf verschiedenen Schichtstrukturen zusammengesetzt, die in getrennten Fertigungsverfahren erzeugt werden müssen, bevor sie zu der Mikropumpe vereinigt werden können. Sie erfordert daher einen hohen Herstellungsaufwand. Ferner hat sie vergleichsweise große Abmessungen und trotz einer niedrigen Förderleistung in der Größenordnung von wenigen Mikrolitern pro Minute eine Leistungsaufnahme in der Größenordnung von etwa 2 Watt.

Aus der Fachveröffentlichung M. Esashi, et al., " Normally Closed Microvalve and Micropump Fabricated on a Silicon Wafer", Sensors and Actuators, 20, 1989, Seiten 163 bis 169 ist eine Membranmikropumpe bekannt, die nach einem von den Wirkungsprinzipien der oben erläuterten Mikropumpe abweichenden piezoelektrischen Wirkungsprinzip arbeitet. Die bekannte Pumpe umfaßt im wesentlichen ein piezoelektrisches Betätigungsteil, das mit einer Membranstruktur über Klebestellen verbunden ist, die ihrerseits mit einer plattenförmigen Ventilsitzglasstruktur verbunden ist. Diese bekannte Pumpe erfordert nicht nur hohe Betriebsspannungen in der Größenordnung von 100 V zum Ansteuern des piezoelektrischen Betätigungselementes, sondern hat mit Maßen von 20x20x10 mm einen für viele Anwendungsfälle zu hohen Raumbedarf.

Aus der Fachveröffentlichung T. Ohnstein, "Micromachined Silicon Microvalve", Proceedings IEEE, 11.-14. Februar 1990, Napa Valley, Kalifornien, USA, ist bereits ein elektrostatisch betätigbares Siliziummikroventil zum elektrisch steuerbaren Modulieren eines Gasflusses bekannt. Das bekannte Mikroventil besteht aus einer Siliziumgrundplatte mit einer Einlaßöffnung, auf der eine dielektrische Schicht angeordnet ist, die in eine bewegliche Verschlußplatte übergeht. Die Verschlußplatte umschließt eine erste Elektrodenfläche, der eine zweite Elektrodenfläche innerhalb der dielektrischen Schicht gegenüberliegt. Durch Anlegen einer geeigneten Steuerspannung kann das normalerweise geöffnete Ventil in einen geschlossenen Zustand gebracht werden.

Gegenüber diesem Stand der Technik liegt der vorliegenden Erfindung die Aufgabe zugrunde, eine mikrominiaturisierte Pumpe der eingangs genannten Art so weiterzubilden, daß sie bei einem hohen möglichen Miniaturisierungsgrad und niedriger Leistungsaufnahme mit für die Großserienherstellung geeigneten Technologien herstellbar ist.

Diese Aufgabe wird bei einer mikrominiaturisierten Pumpe nach dem Oberbegriff des Patentanspruchs 1 durch die im kennzeichnenden Teil des Patentanspruchs 1 angegebenen Merkmale gelöst.

Bei der erfindungsgemäßen mikrominiaturisierten Pumpe weist wenigstens einer der beiden Pumpenkörper einen membranartig dünnen, elastischen Bereich auf, innerhalb dessen zumindest ein Rückschlagventil, vorzugsweise eine Vielzahl von Rückschlagventilen angeordnet ist. Jedes Rückschlagventil ist einstückig mit dem membranartig dünnen, elastischen Bereich ausgebildet und weist eine Eintrittsöffnung sowie eine der Eintrittsöffnung gegenüberliegende, diese überdeckende Verschlußplatte auf, die an einer ihrer Schmalseiten in den membranartig dünnen, elastischen Bereich übergeht. Der membranartig dünne, elastische Bereich umfaßt zumindest einen Teil des elektrisch leitfähigen Bereiches von einem der beiden Pumpenkörper, während der elektrisch leitfähige Bereich des anderen Pumpenkörpers mit einem bezogen auf die Flächenabmessungen des membranartig dünnen, elastischen Bereiches geringen Abstand zu diesem angeordnet ist. Die so geschaffene, integrierte Membran-Ventil-Pumpenstruktur der erfindungsgemäßen mikrominiaturisierten Pumpe kann mit Herstellungstechniken der Halbleitertechnologie erzeugt werden.

Ein besonderer Vorteil der erfindungsgemäßen mikrominiaturisierten Pumpe liegt darin, daß sie bei rückseitiger elektrischer Isolation von wenigstens einem der beiden Pumpenkörper zum Pumpen und Dosieren wässriger oder leitfähiger Lösungen und Flüssigkeiten, wie sie insbesondere im medizinischen und biotechnologischen Bereich auftreten, verwendet werden kann. Daher kommt der erfindungsgemäßen Pumpe besondere Bedeutung bei der inkorporalen Anwendung zur Dosierung beliebiger flüssiger Medikamente zu, die durch den erfindungsgemäß erreichten Grad der Mikrominiaturisierung der erfindungsgemäßen Pumpe erstmals möglich ist.

Ein weiterer bedeutender Vorteil der erfindungsgemäßen mikrominiaturisierten Pumpe liegt darin, daß sie aufgrund ihres Herstellungsverfahrens auch die Integration mit Sensoren und elektrischen Steuerelementen zu einem Mikrosystem erlaubt.

Bevorzugte Weiterbildungen der erfindungsgemäßen mikrominiaturisierten Pumpe sind in den Unteransprüchen angegeben.

Nachfolgend werden unter Bezugnahme auf die beiliegenden Zeichnungen bevorzugte Ausführungsbeispiele der vorliegenden Erfindung näher erläutert. Es zeigen:
- Fig. 1: eine Vertikalschnittdarstellung einer ersten Ausführungsform der erfindungsgemäßen mikrominiaturisierten Pumpe;
- Fig. 2: eine perspektivische Darstellung einer Ventilstruktur innerhalb der Membran, wie sie in der Pumpe gemäß Fig. 1 mehrfach enthalten ist; und
- Fig. 3 bis 6: Vertikalschnittdarstellungen einer zweiten bis fünften Ausführungsform der erfindungsgemäßen mikrominiaturisierten Pumpe.

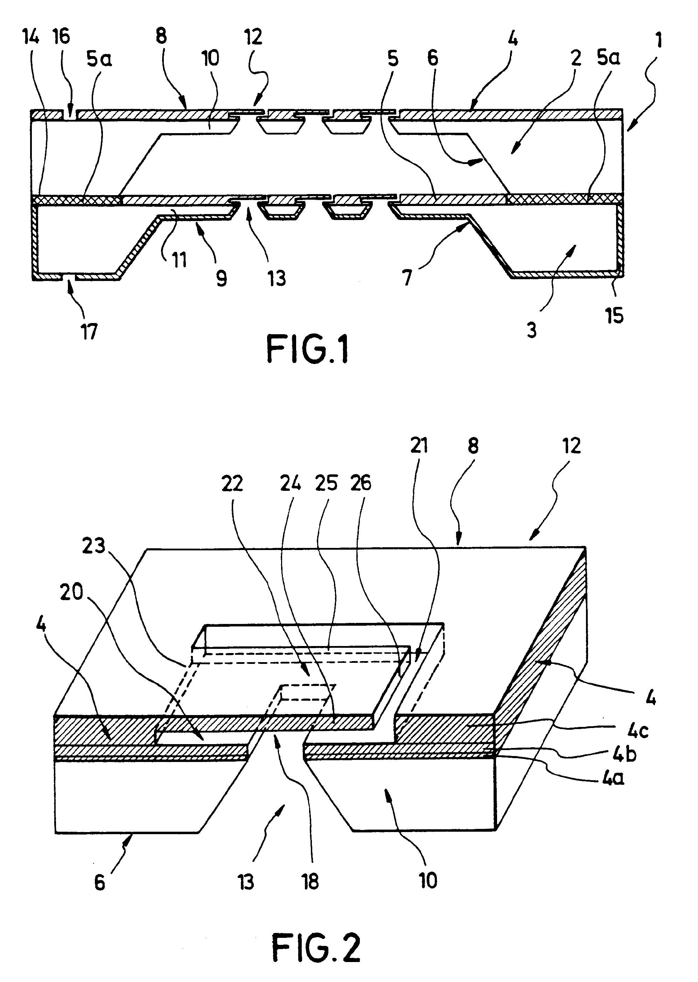

Das in Fig. 1 gezeigte Ausführungsbeispiel der erfindungsgemäßen mikrominiaturisierten Pumpe zum Pumpen von Flüssigkeiten oder Gasen ist in seiner Gesamtheit mit dem Bezugszeichen 1 bezeichnet und umfaßt im wesentlichen einen ersten Pumpenkörper 2, der oberhalb eines zweiten Pumpenkörpers 3 angeordnet und fest mit diesem verbunden ist. Beide Pumpenkörper 2, 3 bestehen aus Silizium. Auf der Vorderseite der Pumpenkörper 2, 3 ist eine Siliziumnitridschicht 4, 5, die eine Dicke von etwa 1 bis 5 Mikrometer hat. Jeder Pumpenkörper 2, 3 weist eine rückseitige Ausnehmung 6, 7 auf, die durch fotolithographisches Festlegen einer rückseitigen Ätzöffnung und anschließendes anisotropes Ätzen erzeugt wird. Wie an sich bekannt ist, kann ein derartiges anisotropes Ätzen in Silizium beispielsweise mittels einer 30 %igen KOH-Lösung vorgenommen werden. Durch die rückseitigen, pyramidenstumpfförmigen Ausnehmungen 6, 7 werden membranartig dünne, elastische Bereiche 8, 9 innerhalb der Pumpenkörper 2, 3 festgelegt. Die mebranartig dünnen, elastischen Bereiche bestehen bei dem gezeigten Ausführungsbeispiel aus einer Siliziummembran 10, 11 mit der darüberliegenden Siliziumnitridschicht 4, 5.

Vorzugsweise umfaßt jede der nachfolgend als Siliziumnitridschicht 4, 5 bezeichneten Schichten eine Schichtenfolge aus einer thermischen Oxidschicht 4a von 100 bis 300 nm Dicke, einer ersten Siliziumnitridteilschicht 4b von 400 bis 1000 nm Dicke und einer zweiten Siliziumnitridteilschicht 4c von 400 bis 2000 nm Dicke, wie dies insbesondere in Fig. 2 zu sehen ist.

Innerhalb eines jeden membranartig dünnen, elastischen Bereiches 8, 9 liegt eine Mehrzahl von feldförmig angeordneten Rückschlagventilen 12, 13, deren Struktur später unter Bezugnahme auf Fig. 2 näher erläutert wird.

Die Rückseite des ersten Pumpenkörpers 2 ist mit einer vorderseitigen Oxidschicht 5a des zweiten Pumpenkörpers 3 mittels einer Pyrexzwischenschicht 14 durch elektrostatisches Bonden verbunden.

Ebenfalls können die Pumpenkörper 2, 3 miteinander verklebt werden. Der zweite Pumpenkörper 3 weist zum Zwecke seiner elektrischen Isolation in Richtung zu seiner Rückseite hin eine thermische Oxidschicht 15 auf. Diese thermische Oxidschicht 15 bildet zusammen mit der Siliziumnitridschicht 5 des zweiten Pumpenkörpers 3 eine diesen vollständig umschließende elektrische Isolation. Der erste Pumpenkörper 2 wird über einen ersten Ohm'schen Kontakt 16 und der zweite Pumpenkörper 3 über einen zweiten Ohm'schen Kontakt 17 mit den beiden Potentialen einer Wechselspannung beaufschlagt. Aufgrund der durch die angelegte Wechselspannung erzeugten elektrostatischen Kräfte werden die membranartig dünnen, elastischen Bereiche 8, 9 der Pumpe 1 in entgegengesetzte Schwingungen versetzt. Vorzugsweise ist die Frequenz der angelegten Wechselspannung derart gewählt, daß sie der ersten mechanischen Grundschwingung der beiden membranartigen Bereiche 8, 9 entspricht. Durch geeignete Wahl der Frequenz und des Spannungswertes kann der Membranhub und somit die Pumpmenge der pumpe 1 exakt eingestellt werden.

Fig. 2 zeigt eine perspektivische Darstellung eines der bei der Pumpe 1 gemäß Fig. 1 angeordneten Rückschlagventiles 12. Wie in Fig. 2 im einzelnen zu sehen ist, liegt das Rückschlagventil 12 innerhalb des membranartig dünnen, elastischen Bereiches 8, der die Siliziumnitridschicht 4 und die Siliziummembran 10 umfaßt. Das Ventil 12 hat eine in der untersten Ebene der Siliziumnitridschicht 4 angeordnete Einlaßöffnung 18 mit einem Querschnitt von beispielsweise 25 x 60 Mikrometer. Die Eintrittsöffnung 18 steht über eine durch anisotropes Ätzen erzeugte rückseitige Aussparung 19 mit der rückseitigen Ausnehmung 6 des ersten Pumpenkörpers 2 in Verbindung.

In einer Mittenebene der Siliziumnitridschicht 4 liegt eine Flächenausnehmung 20, die zentrisch zu der Eintrittsöffnung 18 angeordnet ist. Eine in Draufsicht auf den membranartig dünnen, elastischen Bereich 8 im wesentlichen U-förmige Randausnehmung 21 erstreckt sich in vertikaler Richtung nach unten bis zur Flächenausnehmung 20, wodurch eine Verschlußplatte 22 definiert wird, die an einer Schmalseite 23 einstückig in die Siliziumnitridschicht 4 übergeht, während die übrigen Schmalseiten 24, 25, 26 von der Randausnehmung 21 umgrenzt sind. Somit liegt die Verschlußplatte 22 in einem geringen Abstand von beispielsweise 1 Mikrometer oberhalb der Eintrittsöffnung 18 zentrisch über dieser. Die Verschlußplatte 22 bildet zusammen mit der Eintrittsöffnung 18 aufgrund der Eigenelastizität der Verschlußplatte 22 ein Rückschlagventil 12, da sich die Verschlußplatte 22 bei einem von der Rückseite wirkenden Fluiddruck von der Eintrittsöffnung 18 eigenelastisch wegbiegen läßt und somit diese freigibt, während bei einem von der Vorderseite der Nitridschicht 4 wirkenden Fluiddruck die Verschlußplatte 22 oberhalb der Eintrittsöffnung 18 zur Anlage kommt und somit diese verschließt.

Für die Herstellung der in den Fig. 1 und 2 gezeigten erfindungsgemäßen Pumpe 1 wird zunächst auf die Oxidschicht 4a des thermisch oxidierten Siliziumwafers die erste Nitridschicht 4b aufgebracht, in die in einem fotolithographischen Ätzverfahren eine fensterförmige Ausnehmung zum Festlegen der Eintrittsöffnung 18 geätzt wird. Mittels einer geeigneten Maske wird in die Eintrittsöffnung 18 und in den Bereich der späteren Flächenausnehmung 20 eine Opferschicht von 400 bis 2000 nm, vorzugsweise 1000 nm Dicke eingebracht, die beispielsweise aus PBSG oder einem Metall bestehen kann. Diese Struktur wird mit der zweiten Nitridschicht 4c, die in Fig. 2 angedeutet ist, überdeckt. In einem weiteren fotolithographischen Schritt wird die Randausnehmung 21 definiert und geätzt. Die Opferschicht wird in einem weiteren Ätzschritt mittels eines selektiven Ätzprozesses entfernt, wobei die Nitridschicht nicht angegriffen wird. Anschließend werden durch zwei aufeinanderfolgende Fotolithographie- und Ätz-Schritte zunächst die rückseitige Ausnehmung 6 und anschließend die rückseitigen Aussparungen 19 festgelegt und freigeätzt. Falls eine Isolation des Halbleiterpumpenkörpers gewünscht ist, kann dies nun durch thermisches Oxidieren des Siliziums geschehen. Gleichfalls kommt eine Isolation mittels chemischen Dampfabscheiden oder Aufsputtern eines Isolatormaterials in Betracht.

Der Verfahrensschritt des selektiven Ätzens der Opferschicht kann in Abweichung von dem obigen Verfahren auch nach dem Ätzen der rückseitigen Aussparung 19 und der Eintrittsöffnung 18 erfolgen.

Durch Aufbringen einer Pyrexzwischenschicht 14 wird die Rückseite des ersten Pumpenkörpers 2 mit der vorderseitigen Siliziumnitridschicht 5 des zweiten Pumpenkörpers 3 mittels elektrostatischen Bondens verbunden.

Für einen Betrieb der erfindungsgemäßen elektrostatischen Pumpe mit der in Fig. 1 gezeigten Struktur mit einer niedrigen Wechselspannung ist es erforderlich, daß der gegenseitige Abstand der beiden membranartigen Bereiche 8, 9 sehr viel geringer ist als die Lateralabmessung des membranartigen Bereiches 8, 9. Üblicherweise haben Siliziumwafer eine Dicke von etwa 500 Mikrometer. Um die in Fig. 1 gezeigte Ausführungsform der erfindungsgemäßen Pumpe 1 mit einer niedrigen Spannung betreiben zu können, ist es jedoch erforderlich, zumindest die Dicke des ersten Pumpenkörpers 2 auf vorzugsweise 5 bis 20 Mikrometer zu reduzieren.

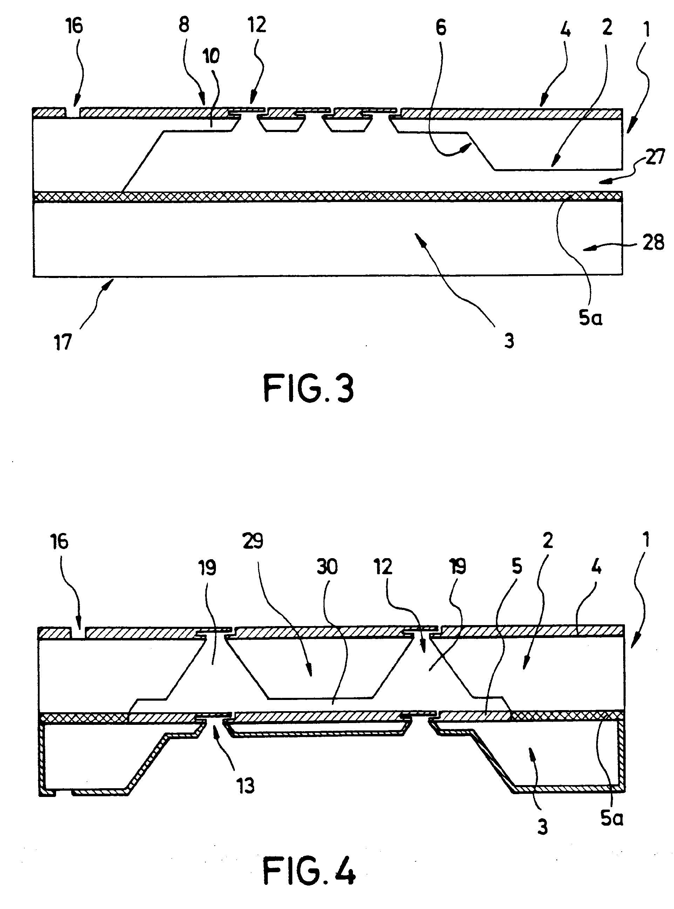

Nachfolgend werden unter Bezugnahme auf die Fig. 3 bis 6 eine zweite bis fünfte Ausführungsform der erfindungsgemäßen pumpe näher erläutert. Diese Ausführungsbeispiele stimmen bis auf die nachfolgend erläuterten Abweichungen mit dem Ausführungsbeispiel nach den Fig. 1 und 2 überein, so daß auf eine nochmalige Erläuterung gleicher oder ähnlicher Teile oder Herstellungsverfahren verzichtet werden kann.

Bei der zweiten Ausführungsform der erfindungsgemäßen Pumpe gemäß Fig. 3 stimmt der erste Pumpenkörper 2 mit Ausnahme eines an seiner Unterseite angeordneten Seitenkanales mit dem ersten Pumpenkörper 2 der ersten Ausführungsform gemäß Fig. 1 bzw. Fig. 2 identisch überein.

Der zweite Pumpenkörper 3 ist jedoch hier als ebener Siliziumkörper 28 mit darauf angeordneter Oxide-Pyrex-Schicht 5a ausgebildet und hat lediglich die Funktion einer Gegenelektrode für den membranartigen Bereich 8 des ersten Pumpenkörpers 2. Für den Fachmann ist es offensichtlich, daß es zur Erzielung einer Pumpwirkung zumindest dann nicht eines zweiten Satzes von Rückschlagventilen bedarf, wenn mit der Pumpe eine Flüssigkeit gefördert wird, die bei einem oszillierenden Schwingen des membranartig dünnen, elastischen Bereiches 8 des ersten Pumpenkörpers 2 aufgrund ihrer Massenträgheit in einem kontinuierlich fließenden Zustand gehalten wird.

Bei der dritten Ausführungsform gemäß Fig. 4 stimmt hingegen der zweite Pumpenkörper 3 im wesentlichen mit demjenigen 3 des ersten Ausführungsbeispieles gemäß Fig. 1 überein. Jedoch ist bei dem dritten Ausführungsbeispiel der erfindungsgemäßen Pumpe 1 gemäß Fig. 4 der membranartig dünne, elastische Bereich 8 des ersten Pumpenkörpers 2 durch einen starren Gegenelektrodenbereich 29 ersetzt, der dadurch definiert ist, daß bei dem hier gezeigten Ausführungsbeispiel die rückseitige Ausnehmung 30 mittels eines anisotropen Ätzverfahrens lediglich um den gewünschten Elektrodenabstand von 5 bis 20 Mikrometer gegenüber der Rückseite des Siliziumwafers des ersten Pumpenkörpers 2 eingeätzt wird. Hierdurch ist es möglich, den Siliziumwafer 2 in seiner ursprünglichen Dicke von etwa 500 Mikrometern zu belassen, da die Dicke des Siliziumwafers bei dieser Ausführungsform unabhängig von der Größe des Elektrodenabstandes ist.

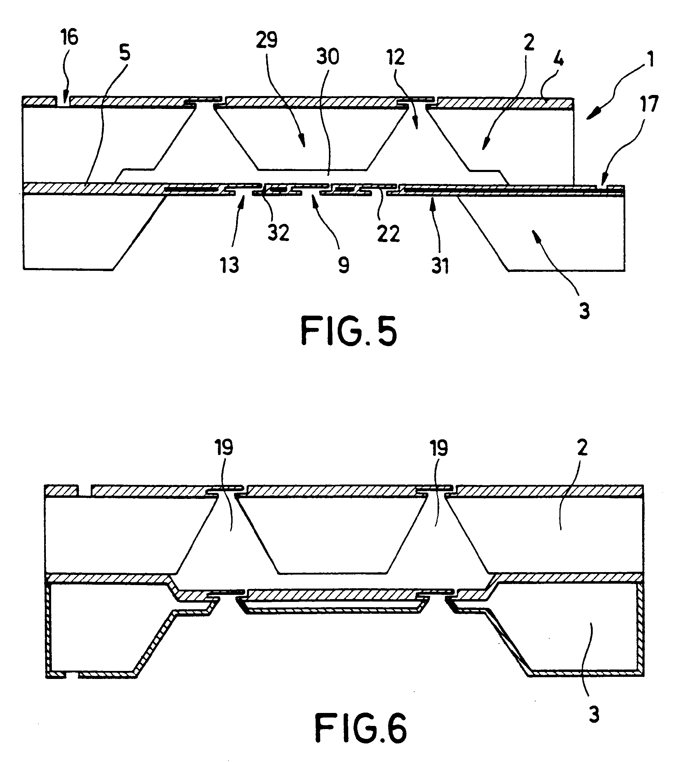

Die Ausführungsform gemäß Fig. 5, die das vierte Ausführungsbeispiel der erfindungsgemäßen Pumpe 1 darstellt, stimmt bezüglich des ersten Pumpenkörpers identisch mit dem dritten Ausführungsbeispiel gemäß Fig. 4 überein. Der zweite Pumpenkörper 3 besteht in seinem membranartig dünnen, elastischen Bereich 9 ausschließlich aus der Siliziumnitridschicht 5, in die eine Metallelektrodenschicht 31 in der Weise eingebettet ist, das diese allseits von der Siliziumnitridschicht 5 umschlossen ist. Die Metallelektrodenschicht innerhalb der Siliziumnitridschicht 5 hat im Bereich der Rückschlagventile 13 Elektrodenausnehmungen 32, damit die Bewegung der Verschlußplatten 22 unabhängig von der angelegten Steuerspannung ist.

Die fünfte Ausführungsform der erfindungsgemäßen Pumpe 1 unterscheidet sich dadurch von der vierten Ausführungsform gemäß Fig. 5, daß die dem zweiten Pumpenkörper 3 zugewandte Rückseite des ersten Pumpenkörpers 2 im wesentlichen eben ausgebildet ist, wenn man von den rückseitigen Aussparungen 19 absieht. Anstelle der rückseitigen Abstandsausnehmung 30 des ersten Pumpenkörpers 2 bei dem dritten Ausführungsbeispiel gemäß Fig. 4 weist hier der zweite Pumpenkörper 3 eine durch anisotropes Ätzen erzeugte vorderseitige Abstandsausnehmung 33 auf, durch die der membranartig dünne, elastische Bereich 9 des zweiten Pumpenkörpers um den gewünschten Elektrodenabstand in Richtung zu dessen Rückseite versetzt ist. Ebenso wie bei der Ausführungsform gemäß Fig. 4 wird bei der fünften Ausführungsform gemäß Fig. 6 der Vorteil erreicht, daß die Waferdicke unabhängig von dem gewünschten Elektrodenabstand ist.

In Abweichung zu den in den Fig. 1 bis 6 gezeigten Pumpenstrukturen können bei hohen Pumpdrucken oder bei einer gegebenenfalls für erforderlich erachteten Reduktion der Druckbeaufschlagung der Ventile mehr als zwei Pumpenkörper übereinander angeordnet werden.

Anstelle des als bevorzugt angesehenen Siliziums als Ausgangsmaterial für sämtliche Pumpenkörper ist es möglich, lediglich einen Pumpenkörper aus Silizium oder einem anderen Halbleitermaterial zu fertigen, und den anderen Pumpenkörper aus einem geeigneten anderen Material herzustellen. Insbesondere bei der Ausführungsform gemäß Fig. 3 kann jedes als Elektrode geeignete Material für den zweiten Pumpenkörper 3 eingesetzt werden.

Bei den gezeigten Ausführungsbeispielen wurde lediglich der leitfähige Halbleiterbereich von einem 3 der beiden Pumpenkörper 2, 3 durch eine vollständig einschließende Isolationsschicht 5, 15 elektrisch gegenüber dem zu pumpenden Fluid isoliert. Falls eine vollständige Potentialtrennung gewünscht ist, kann selbstverständlich auch der zweite Pumpenkörper in entsprechender Weise isoliert werden.

## Patentansprüche

1. Mikrominiaturisierte Pumpe
mit zumindest einem ersten und einem zweiten Pumpenkörper (2, 3), die übereinander angeordnet und miteinander verbunden sind,
wobei zumindest einer (2) der Pumpenkörper (2, 3) wenigstens teilweise aus einem Halbleitermaterial besteht, und
wobei beide Pumpenkörper (2, 3) elektrisch leitfähige Bereiche umfassen, die gegeneinander elektrisch isoliert sind,
dadurch gekennzeichnet,
daß wenigstens einer der beiden Pumpenkörper (2, 3) einen membranartig dünnen, elastischen Bereich (8, 9) aufweist,
daß wenigstens ein Rückschlagventil (12, 13) innerhalb des membranartig dünnen, elastischen Bereiches (8, 9) angeordnet ist, das integriert mit dem membranartig dünnen, elastischen Bereich (8, 9) ausgebildet ist und eine Eintrittsöffnung (18) sowie eine die Eintrittsöffnung überdeckende Verschlußplatte (22) aufweist, die an einer (23) ihrer Schmalseiten (23, 24, 25, 26) in den membranartig dünnen, elastischen Bereich übergeht,
daß der membranartig dünne, elastische Bereich (8, 9) zumindest einen Teil des elektrisch leitfähigen Bereiches des Pumpenkörpers umfaßt, und
daß der elektrisch leitfähige Bereich des anderen Pumpenkörpers mit einem bezogen auf die Flächenabmessungen des membranartig dünnen, elastischen Bereiches (8, 9) geringen Abstand zu diesem angeordnet ist.

2. Mikrominiaturisierte Pumpe nach Anspruch 1, dadurch gekennzeichnet,
daß der elektrisch leitfähige Bereich von wenigstens einem der Pumpenkörper (2, 3) allseitig von einer elektrisch isolierenden Schicht (5, 15) umschlossen ist.

3. Mikrominiaturisierte Pumpe nach Anspruch 1 oder 2, dadurch gekennzeichnet,
daß der erste, den membranartig dünnen, elastischen Bereich (8) aufweisende und teilweise aus einem Halbleitermaterial bestehende Pumpenkörper (2) eine vorderseitige, dielektrische Schicht (4), in der das Rückschlagventil ausgebildet ist, und eine rückseitige, durch anisotropes Ätzen erzeugte Ausnehmung (6) aufweist, die den membranartig dünnen, elastischen Bereich (8) definiert.

4. Mikrominiaturisierte Pumpe nach Anspruch 3, dadurch gekennzeichnet,
daß der erste Pumpenkörper (2) ferner eine vorderseitige, durch anisotropes Ätzen erzeugte Abstandsausnehmung (33) aufweist, die zusammen mit der rückseitigen Ausnehmung (6) den membranartig dünnen, elastischen Bereich (9) definiert, und
daß die vorderseitige, dielektrische Schicht (5) in der vorderseitigen Abstandsausnehmung (33) angeordent ist.

5. Mikrominiaturisierte Pumpe nach Anspruch 3 oder 4,
dadurch gekennzeichnet,
daß die dielektrische Schicht (4, 5) eine Siliziumnitridschicht oder eine Schicht aus thermischem Oxid und Siliziumnitrid ist.

6. Mikrominiaturisierte Pumpe nach einem der Ansprüche 3 bis 5, dadurch gekennzeichnet,
daß die Eintrittsöffnung (18) des Rückschlagventiles (12, 13) in einer von der Vorderseite des membranartig dünnen, elastischen Bereiches (8, 9) beabstandeten Ebene der dielektrischen Schicht (4, 5) liegt, und
daß die Eintrittsöffnung (18) mit der rückseitigen Ausnehmung (6) über eine durch anisotropes Ätzen erzeugte rückseitige Aussparung (19) verbunden ist.

7. Mikrominiaturisierte Pumpe nach Anspruch 6, dadurch gekennzeichnet,
daß eine Flächenausnehmung (20) zwischen der Verschlußplatte (22) und der Eintrittsöffnung (18) vorgesehen ist, durch die die Verschlußplatte (22) von der Eintrittsöffnung (18) beabstandet ist, und
daß eine in Draufsicht auf den membranartig dünnen, elastischen Bereich (8, 9) im wesentlichen U-förmige Randausnehmung (21), die sich bis zu der Flächenausnehmung (20) erstreckt, diejenigen Schmalseiten (24, 25, 26) der Verschlußplatte (22) definiert, an denen diese nicht in den membranartig dünnen, elastischen Bereich (8, 9) übergeht.

8. Mikrominiaturisierte Pumpe nach einem der Ansprüche 3 bis 7 in direkter oder indirekter Rückbeziehung auf Anspruch 2, dadurch gekennzeichnet,
daß die den elektrisch leitfähigen Bereich allseitig umschließende elektrisch isolierende Schicht einerseits durch die vorderseitige, dielektrische Schicht (5) und andererseits durch eine rückseitige Isolatorschicht (15) gebildet wird.

9. Mikrominiaturisierte Pumpe nach Anspruch 8, dadurch gekennzeichnet,
daß die rückseitige Isolatorschicht durch ein thermisches Oxid des Halbleitermaterials des Pumpenkörpers (2, 3) gebildet ist.

10. Mikrominiaturisierte Pumpe nach Anspruch 8, dadurch gekennzeichnet,
daß die rückseitige Isolatorschicht durch einen chemisch im Dampfabscheidungsverfahren auf die Rückseite des Pumpenkörpers (2, 3) aufgebrachten Isolator gebildet ist.

11. Mikrominiaturisierte Pumpe nach Anspruch 8, dadurch gekennzeichnet,
daß die rückseitige Isolatorschicht durch ein mittels Sputtern auf die Rückseite des Pumpenkörpers (2, 3) aufgebrachtes Isolatormaterial gebildet ist.

12. Mikrominiaturisierte Pumpe nach einem der Ansprüche 1 bis 11, dadurch gekennzeichnet,
daß die beiden Pumpenkörper (2, 3) im wesentlichen aus Silizium bestehen.

13. Mikrominiaturisierte Pumpe nach einem der Ansprüche 1 bis 12, dadurch gekennzeichnet,
daß die beiden Pumpenkörper (2, 3) durch eine Pyrexzwischenschicht (14) mittels elektrostatischen Bondens miteinander verbunden sind.

14. Mikrominiaturisierte Pumpe nach einem der Ansprüche 4 bis 13 in direkter oder indirekter Rückbeziehung auf Anspruch 3, dadurch gekennzeichnet,
daß der leitfähige Bereich eines Pumpenkörpers (2, 3) durch eine in die dielektrische Schicht (5) eingebettete Metallelektrodenschicht (31) gebildet ist, die im Bereich der Ventile (12, 13) Elektrodenausnehmungen (32) hat.

15. Mikrominiaturisierte Pumpe nach einem der Ansprüche 1 bis 14, dadurch gekennzeichnet,
daß der eine Pumpenkörper einen membranartig dünnen, elastischen Bereich (9) aufweist, und
daß sich der leitfähige Bereich des anderen Pumpenkörpers (2) bis nahe an den membranartig dünnen, elastischen Bereich (9) des einen Pumpenkörpers (3) erstreckt.

16. Mikrominiaturisierte Pumpe nach Anspruch 15, dadurch gekennzeichnet,
daß sich die dielektrische Schicht (5) des einen Pumpenkörpers als Ebene über dessen Fläche erstreckt, und
daß der andere Pumpenkörper (2) auf seiner dem einen Pumpenkörper (3) zugewandten Rückseite eine durch anisotropes Ätzen erzeugte Abstandsausnehmung (30) aufweist.

17. Mikrominiaturisierte Pumpe nach Anspruch 15 in direkter oder indirekter Rückbeziehung auf Anspruch 4, dadurch gekennzeichnet,
daß der andere Pumpenkörper (2) auf seiner dem einen Pumpenkörper (3) zugewandten Rückseite im wesentlichen eben ausgebildet ist, und
daß die vorderseitige Ausnehmung (33) des einen Pumpenkörpers (3) einen Abstand des membranartig dünnen, elastischen Bereiches (9) zu der Rückseite des anderen Pumpenkörpers (2) festlegt.

## Claims

1. A microminiaturized pump comprising
at least one first and one second pump body (2, 3), which are arranged one on top of the other and which are interconnected,
at least one (2) of said pump bodies (2, 3) consisting at least partially of a semiconductor material, and
both pump bodies (2, 3) including electrically conductive regions, which are electrically insulated from one another,
characterized in that
at least one of the two pump bodies (2, 3) has a thin, flexible, diaphragm-like region (8, 9),
that at least one check valve (12, 13) is arranged within said thin, flexible, diaphragm-like region (8, 9), said check valve being formed integrally with said thin, flexible, diaphragm-like region (8, 9) and having an inlet aperture (18) as well as a cover plate (22), which covers said inlet aperture and which merges with said thin, flexible, diaphragm-like region at one (23) of its narrows sides (23, 24, 25, 26),
that said thin, flexible, diaphragm-like region (8, 9) includes at least part of the electrically conductive region of the pump body, and
that, in relation to the surface dimensions of the thin, flexible, diaphragm-like region (8, 9), the electrically conductive region of the other pump body is arranged at a short distance from said thin, flexible, diaphragm-like region.

2. A microminiaturized pump according to claim 1,
characterized in that the electrically conductive region of at least one of the pump bodies (2, 3) is enclosed by an electrically insulating layer (5, 15) on all sides.

3. A microminiaturized pump according to claim 1 or 2,
characterized in that the first pump body (2), which includes the thin, flexible, diaphragm-like region (8) and which partially consists of a semiconductor material, is provided with a front dielectric layer (4), in which the check valve is formed, and with a rear recess (6) produced by anisotropic etching and defining the thin, flexible diaphragm-like region (8).

4. A microminiaturized pump according to claim 3,
characterized in that the first pump body (2) is additionally provided with a front spacing recess (33), which is produced by anisotropic etching and which defines the thin, flexible, diaphragm-like region (9) together with the rear recess (6), and
that the front dielectric layer (5) is arranged in said front spacing recess (33).

5. A microminiaturized pump according to claim 3 or 4,
characterized in that the dielectric layer (4, 5) is a layer of silicon nitride or a layer of thermal oxide and silicon nitride.

6. A microminiaturized pump according to one of the claims 3 to 5,
characterized in that the inlet aperture (18) of the check valve (12, 13) is positioned in a plane of the dielectric layer (4, 5) which extends in spaced relationship with the front side of the thin, flexible, diaphragm-like region (8, 9), and
that the inlet aperture (18) communicates with the rear recess (6) via a rear recess (19) produced by anisotropic etching.

7. A microminiaturized pump according to claim 6,
characterized in that a planar recess (20) is provided between the cover plate (22) and the inlet aperture (18), said cover plate (22) being spaced from said inlet aperture (18) by means of said planar recess (20), and
that a boundary recess (21), which, when seen in a top view of the thin, flexible, diaphragm-like region (8, 9), is essentially U-shaped and which extends down to the planar recess (20), defines the narrow sides (24, 25, 26) of the cover plate (22) at which said cover plate does not merge with said thin, flexible, diaphragm-like region (8, 9).

8. A microminiaturized pump according to one of the claims 3 to 7 depending directly or indirectly on claim 2,
characterized in that the electrically insulating layer enclosing the electrically conductive region on all sides is formed, on the one hand, by said front dielectric layer (5) and, on the other hand, by a rear insulating layer (15).

9. A microminiaturized pump according to claim 8,
characterized in that the rear insulating layer is formed by a thermal oxide of the semiconductor material of the pump body (2, 3).

10. A microminiaturized pump according to claim 8,
characterized in that the rear insulating layer is formed by an insulator, which is chemically applied to the back of the pump body (2, 3) in a vapour deposition process.

11. A microminiaturized pump according to claim 8,
characterized in that the rear insulating layer is formed by an insulating material applied to the back of the pump body (2, 3) by means of sputtering.

12. A microminiaturized pump according to one of the claims 1 to 11,
characterized in that both pump bodies (2, 3) consist essentially of silicon.

13. A microminiaturized pump according to one of the claims 1 to 12,
characterized in that the two pump bodies (2, 3) are interconnected via an intermediate Pyrex layer (14) by means of electrostatic bonding.

14. A microminiaturized pump according to one of the claims 4 to 13 depending directly or indirectly on claim 3,
characterized in that the conductive region of one pump body (2, 3) is formed by a metal electrode layer (31) embedded in the dielectric layer (5) and provided with electrode openings (32) in the area of the valves (12, 13).

15. A microminiaturized pump according to one of the claims 1 to 14,
characterized in that one of the pump bodies is provided with a thin, flexible, diaphragm-like region (9), and
that the conductive region of the other pump body (2) extends up to a point located close to said thin, flexible, diaphragm-like region (9) of said first-mentioned pump body (3).

16. A microminiaturized pump according to claim 15,
characterized in that the dielectric layer (5) of one pump body extends as a plane over the surface thereof, and
that the other pump body (2) is provided with a spacing recess (30), which is produced by anisotropic etching and which is located at the pump body back facing the pump body (3),

17. A microminiaturized pump according to claim 15 depending directly or indirectly on claim 4,
characterized in that, at its back facing the pump body (3), the other pump body (2) is essentially flat, and
that the front recess (33) of said first-mentioned pump body (3) determines a spacing between the thin, flexible, diaphragm-like region (9) and the back of said other pump body (2).

## Revendications

1. Pompe microminiaturisée
avec au moins un premier et un second corps de pompe (2, 3) disposés l'un au-dessus de l'autre et reccordés l'un à l'autre,
au moins l'un (2) des corps de pompe (2, 3) consistant au moins en partie en un matériau semi-conducteur, et
les deux corps de pompe (2, 3) comportant des zones électriquement conductrices isolées électriquement l'une de l'autre,
caractérisée en ce
qu'au moins l'un des deux corps de pompe (2, 3) présente une zone élastique mince en forme de membrane (8, 9),
qu'au moins un clapet de retenue (12, 13) est disposé à l'intérieur de la zone élastique mince en forme de membrane (8, 9), lequel se présente de manière intégrée avec la zone élastique mince en forme de membrane (8, 9) et présente un orifice d'entrée (18) ainsi qu'une plaque d'obturation (22) recouvrant l'orifice d'entrée, laquelle se confond, à l'un (23) de ses côtés étroits (23, 24, 25, 26) avec la zone élastique mince en forme de membrane,
que la zone élastique mince en forme de membrane (8, 9) comporte au moins une partie de la zone électriquement conductrice du corps de pompe, et
que la zone électriquement conductrice de l'autre corps de pompe est disposée, comparé aux dimensions de surface de la zone élastique mince en forme de membrane (8, 9), à une faible distance de celle-ci.

2. Pompe microminiaturisée suivant la revendication 1, caractérisée en ce que la zone électriquement conductrice d'au moins l'un des corps de pompe (2, 3) est entourée de tous les côtés d'une couche électriquement isolante.

3. Pompe microminiaturisée suivant la revendication 1 ou 2, caractérisée en ce que le premier corps de pompe (2), présentant la zone élastique mince en forme de membrane (8) et réalisé partiellement en un matériau semi-conducteur, présente une couche diélectrique (4) du côté avant, dans laquelle est réalisé le clapet de retenue, et un évidement (6) réalisé par gravure anisotrope, du côté arrière, qui définit la zone élastique mince en forme de membrane (8).

4. Pompe microminiaturisée suivant la revendication 3, caractérisée en ce
que le premier corps de pompe (2) présente, en outre, un évidement d'écartement (33) réalisé par gravure anisotrope qui, avec l'évidement du côté arrière (6), définit la zone élastique mince en forme de membrane (9), et
que la couche diélectrique (5) du côté avant est disposée dans l'évidemment d'écartement du côté avant (33).

5. Pompe microminiaturisée suivant la revendication 3 ou 4, caractérisée en ce que la couche diélectrique (4, 5) est une couche de nitrure de silicium ou une couche d'oxyde thermique et de nitrure de silicium.

6. Pompe microminiaturisée suivant l'une des revendications 3 à 5, caractérisée en ce
que l'orifice d'entrée (18) du clapet de retenue (12, 13) se situe dans un plan de la couche diélectrique (4, 5) distant du côté avant de la zone élastique mince en forme de membrane (8, 9), et que l'orifice d'entrée (18) est relié à l'évidement du côté arrière (6) par un évidement du côté arrière (19) réalisé par gravure anisotrope.

7. Pompe microminiaturisée suivant la revendication 6, caractérisée en ce
qu'il est prévu un évidement de face (20) entre la plaque d'obturation (22) et l'orifice d'entrée (18) par lequel la plaque d'obturation (22) est écartée de l'orifice d'entrée (18), et qu'un évidement de bord (21) sensiblement en forme de "U", en vue de dessus de la zone élastique mince en forme de membrane (8, 9), qui s'étend jusqu'à l'évidement de face (20), définit les côtés étroits (24, 25, 26) de la plaque d'obturation (22) auxquels cette dernière ne se confond pas avec la zone élastique mince en forme de membrane (8, 9).

8. Pompe microminiaturisée suivant l'une des revendications 3 à 7, en relation directe ou indirecte avec la revendication 2, caractérisée en ce que la couche électriquement isolante entourant de tous les côtés la zone élecriquement conductrice est formée, d'une part, par la couche diélectrique du côté avant (5) et, d'autre part, par une couche d'isolateur du côté arrière (15).

9. Pompe microminiaturisée suivant la revendication 8, caractérisée en ce que la couche d'isolateur du côté arrière est formée par un oxyde thermique du matériau semi-conducteur du corps de pompe (2, 3).

10. Pompe microminiaturisée suivant la revendication 8, caractérisée en ce que la couche d'isolateur du côté arrière est formée par un isolateur appliqué sur la face arrière du corps de pompe (2, 3) suivant le procédé de séparation de vapeur.

11. Pompe microminiaturisée suivant la revendication 8, caractérisée en ce que la couche d'isolateur du côté arrière est formée par un matériau isolateur appliqué sur la face arrière du corps de pompe (2, 3) par pulvérisation.

12. Pompe microminiaturisée suivant l'une des revendications 1 à 11, caractérisée en ce que les deux corps de pompe (2, 3) sont essentiellement en silicium.

13. Pompe microminiaturisée suivant l'une des revendications 1 à 12, caractérisée en ce que les deux corps de pompe (2, 3) sont reliés l'un à l'autre par une couche intermédiaire de pyrex, par liaison électrostatique.

14. Pompe microminiaturisée suivant l'une des revendications 4 à 13, en relation directe ou indirecte avec la revendication 3, caractérisée en ce que la zone conductrice d'un corps de pompe (2, 3) est formée par une couche d'électrodes métalliques (31) encastrée dans la couche diélectrique (5) et présentant des évidements d'électrode (32) à l'endroit des soupapes (12, 13).

15. Pompe microminiaturisée suivant l'une des revendications 1 à 14, caractérisée en ce
que l'un des corps de pompe présente une zone élastique mince en forme de membrane (9), et
que la zone conductrice de l'autre corps de pompe (2) s'étend jusqu'à proximité de la zone élastique mince en forme de membrane (9) de l'un des corps de pompe (3).

16. Pompe microminiaturisée suivant la revendication 15, caractérisée en ce
que la couche diélectrique (5) de l'un des corps de pompe s'étend comme un plan au-dessus de cette surface, et
que l'autre corps de pompe (2) présente, sur sa face arrière orientée vers l'un des corps de pompe (3), un évidement d'écartement (30) produit par gravure isotrope.

17. Pompe microminiaturisée suivant la revendication 15, en relation directe ou indirecte avec la revendication 4, caractérisée en ce
que l'autre corps de pompe (2) se présente, sur sa face arrière orientée vers l'un des corps de pompe (3), de forme sensiblement plane, et
que l'évidement du côté avant (33) de l'un des corps de pompe (3) détermine une distance entre la zone élastique mince en forme de membrane (9) et le côté arrière de l'autre corps de pompe (2).
